# EUROPEAN PATENT APPLICATION

(11) **EP 3 335 549 A1**
(43) Date of publication of application: **20.06.2018**
(21) Application number: 16204937.3
(22) Date of filing: 19.12.2016
(51) Int. Cl.: A01K 11/00, A01K 29/00, A61B 5/01, A61B 5/11

(54) **ANIMAL MONITORING SYSTEM AND METHOD**

(71) Applicant: Melexis Technologies SA, 2022 Bevaix (CH)
(72) Inventor: LAUTE, Andreas, 98693 Ilmenau (DE); PEETERS, Walter, 1850 Grimbergen (BE); VAN RANST, Bonifacus, 2870 Puurs (BE); CHOMBAR, Françoise, 3980 Tessenderlo (BE)
(74) Representative: DenK iP

(57) **Abstract**

A subsystem attachable to a living being, comprising: a temperature sensor; a first non-volatile memory for holding an identification information (ID); a second non-volatile memory for holding medical information; an RF transceiver; an LF receiver for measuring signal strength of at least one beacon signal; a control unit being programmed for communicating via the RF transceiver with a readout device for exchanging the identification information and medical information in a first mode of operation; and being programmed for communicating via the RF transceiver with one or more sensor devices for repeatedly receiving sensor information, and for repeatedly retrieving temperature information and signal strength information, and repeatedly transmitting said information via the RF transceiver to a central data collector in a second mode of operation.

## Description

### Field of the invention

The present invention relates to the field of monitoring systems. More in particular the present invention relates to devices and methods for monitoring living beings, in particular animals such as for example cows, to keep track of their physical position and medical health.

### Background of the invention

Wireless sensors for measuring temperature, orientation, strain, acceleration, etc. are known in the art.

Animal monitoring systems where multiple individual sensors are mounted to an animal, and are read out individually by different readout equipment when the animal enters a barn , are also known, but are impractical and expensive.

### Summary of the invention

It is an object of embodiments of the present invention to provide a monitoring system or subsystem and method for monitoring one or more living beings, in particular animals, more specifically cows.

In particular, it is an object of embodiments of the present invention to provide a monitoring system or subsystem and method that is capable of providing sensor information and location information of an animal, moving in a confined environment such as for example in one or more meadows around a farm.

It is an object of particular embodiments of the present invention to provide a monitoring system and method for monitoring a plurality of animals, at least one characteristic and its physical position, which animals can move around in a confined environment such as for example in one or more meadows around a farm.

It is also an object of particular embodiments of the present invention to provide an alarm signal to indicate when an animal is ill.

It is also an object of particular embodiments of the present invention to allow a veterinarian to quickly access the medical history of the animal using said monitoring system.

These and other objectives are accomplished by a system, subsystem, and methods according to embodiments of the present invention.

In a first aspect, the present invention provides a monitoring subsystem attachable to or at least partly implantable in a living being, the subsystem comprising: a temperature sensor; a first non-volatile memory for holding an identification information to be associated to said living being; a second non-volatile memory for holding medical information related to said living being; a wireless transceiver; an LF receiver for receiving at least one beacon signal transmitted by at least one beacon, the LF receiver comprising means for measuring a signal strength of said at least one beacon signal; a control unit connected to the temperature sensor, and to the first and second non-volatile memory and to the wireless transceiver and to the LF receiver, the control unit being programmed for communicating via the wireless transceiver with a readout device for exchanging the identification information and/or for exchanging medical information in a first mode of operation; and the control unit being programmed for repeatedly retrieving temperature information from the temperature sensor, and for repeatedly retrieving signal strength information from the LF receiver indicative of a distance to one or more beacons, and for repeatedly transmitting said temperature information and said signal strength information and said identification information and optionally the medical information via the wireless transceiver to a central data collector in a second mode of operation.

The control unit may be adapted for periodically reading and transmitting said temperature information and signal strength information, with a predefined period, for example in the range from 10 s to 120 s, for example in the range from 20 s to 90 s, for example a period equal to about 30 s or equal to about 45 s or equal to about 60 s or equal to about 75 s.

The first non-volatile memory and the second non-volatile memory may be part of a single non-volatile memory device, which may also include further memory space. Or the first and second non-volatile memory may be two separate memory devices, using different control signals (such as address-lines and/or read/write line).

The second non-volatile memory is adapted for storing at least medical information, but can also be used for storing other information, e.g. non-medical information, such as name, address, contact-information of the owner of the living being, or any other kind of data.

The living being may be an animal.

The living being may be a mammal.

The living being may be a female mammal.

The living being may be a chicken or other poultry.

The living being may be a cow or other cattle.

In an embodiment, the control unit is further programmed for repeatedly communicating via the wireless transceiver with one or more sensor devices attached to or implanted in said living being, for receiving sensor information, and for transmitting said sensor information to the central data collector.

The communication with the sensor device(s) may be bi-directional. The sensor devices may also provide an identification number of their own, to make sure that the local data collector of a particular living being collects information from the same living being, and not from another living being. The sensor device ID(s) are typically also programmed in a non-volatile memory of the identifier device or the local data collector device.

In some embodiments, the sensor information is transmitted only once to the central data collector.

In other embodiments, the sensor information is transmitted at least two times or at least three times to the central data collector. For example, for each transmission to the central data collector, the most recent sensor information can be transmitted, but also one or more historical sensor data, optionally accompanied with a time-stamp. In this way, the risk of "loosing" information, for example due to data collision, is reduced to a minimum.

The "wireless transceiver" may be an RF transceiver. The frequency range used may be in the so called 915 MHz ISM band, and/or in the 2.45 GHz ISM band. The protocol being used may for example be Bluetooth, or RFID, or Zigbee, or any other suitable protocol.

The subsystem may further comprise a timer, and the control unit may be further programmed to go in a low-energy mode during at least 95% of the time, for example during at least 99% of the time, in order to save power.

The first non-volatile memory may be OTP (one time programmable) memory.

It is an advantage of using OTP memory in that it can only be written once, for example shortly after birth of an animal. In this way fraud can be reduced. The OTP memory may be programmed during manufacturing of the subsystem (e.g. module) or in the field, by means of the dedicated reader device, typically operated by a veterinarian. To this end, the control unit may programmed for receiving the identification code from the readout device, and for storing the identification code in the OTP memory, in a third mode of operation.

The first and/or second non-volatile memory, or a non-volatile memory comprising the first and/or second non-volatile memory may be a non-erasable memory.

In an embodiment, the monitoring subsystem is implemented in a single module or capsule.

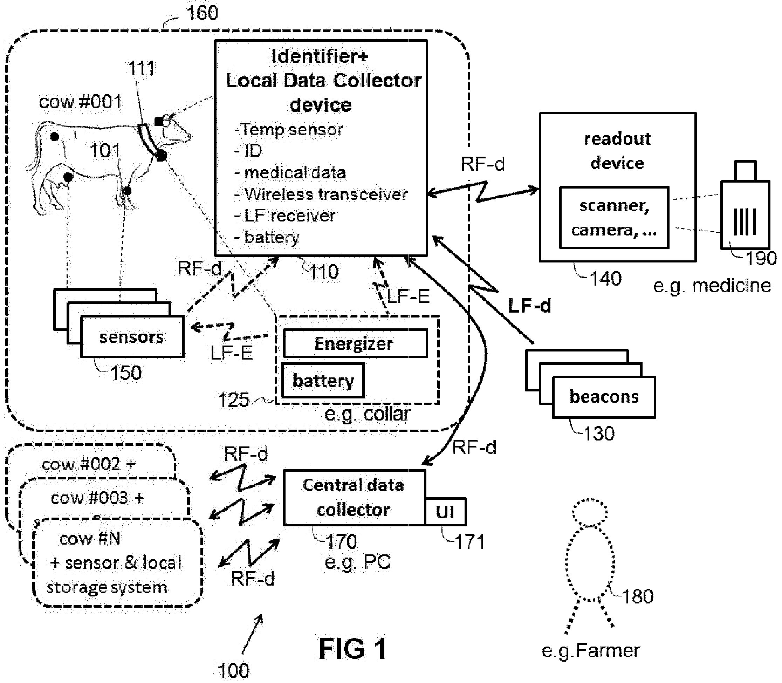

In a first implementation (depicted in FIG. 1), this subsystem is implemented in a single unit or module (e.g. in the form of a capsule comprising a single chip and a battery). This module is referred to herein as "the identifier device", but in this implementation the module also acts as "local data collector" for collecting information from at least one sensor device.

The module may be implantable in the animal, or may be attachable to the animal, for example to one of its ears.

In another embodiment, the monitoring subsystem may be implemented as more than one module, the subsystem comprising at least: a first module comprising at least the temperature sensor, and the first non-volatile memory, and the second non-volatile memory, and the wireless transceiver for communication with the readout device, and for communication with a local data collector if not embedded in the subsystem, and a first local power supply; a second module different from the first module, comprising at least the LF receiver, and a second wireless transceiver for communication with the central data collector and optionally for communication with the one or more sensor devices, and a second local power supply, and optionally an acceleration sensor.

The first local power supply may for example comprise a capacitor and/or a local battery. The second local power supply may for example comprise a capacitor and/or a local battery, irrespective of the first local power supply.

In an embodiment, the monitoring subsystem further comprises: a local energising unit for providing power to the identifier device in a wired or in a wireless manner.

The first and/or second module may further comprise a coil and a rectifier and a capacitor for receiving and storing said power. If the subsystem also comprises one or more sensor devices, the local energising unit may also transmit power to these one or more sensor devices.

In an embodiment, the monitoring subsystem further comprises at least one sensor device attached to or implanted in the living being for measuring a feature or characteristic of the living being.

In an embodiment, the at least one sensor device is a sensor selected from the group consisting of: a strain sensor, a pressure sensor, a milk sensor, a liquid sensor, a PH-sensor, a blood analysis senor, a sugar sensor, a rumination sensor or gas sensor or SCFA sensor; and the at least one sensor comprises a wireless transmitter or a wireless transceiver and is adapted for transmitting sensor information to the subsystem.

In case the living being is a female mammal, the animal may produce milk. The "milk sensor" can be a sensor for measuring the presence of milk, or for measuring a quantity of milk, or for measuring a quality of milk, or for measuring a pH of milk, or any other sensor for measuring a characteristic of milk.

The blood analysis sensor may for example be mounted to a skin of the animal, and detect characteristics of blood based on reflected light.

The at least one sensor would typically also transmit a sensor-ID, along with the sensor data. The at least one sensor would preferably also have a low-power mode, and read-out the sensor element, and transmit the sensed data periodically, and then go to sleep for at least 95% of the time.

In an embodiment, the monitoring subsystem further comprises at least one beacon, preferably a plurality of beacons.

In an embodiment, the monitoring subsystem further comprising the central data collector.

According to a second aspect, the present invention provides a monitoring system comprising: at least one or at least two monitoring subsystems according to the first aspect; at least two beacons; at least one central data collector.

In an embodiment the monitoring subsystem according to the first aspect, or the monitoring system according to the second aspect further comprises a readout device, the readout device further comprising means for obtaining medical information of a medical substance administered to or to be administered to the living being, the readout device being adapted for transmitting said medical information to the subsystem; the monitoring subsystem being further adapted for storing said medical information in the second non-volatile memory.

According to a third aspect, the present invention provides an animal comprising a monitoring subsystem according to the first aspect.

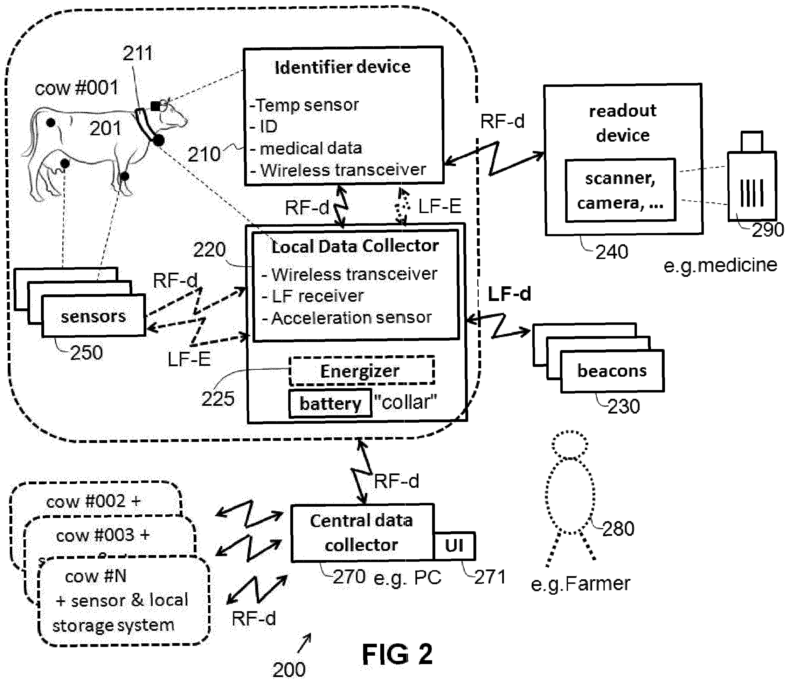

The subsystem may be attached to a skin (on the outside of the skind), or the subsystem may be implanted in the animal, for example under the skin. This is especially desirable for the "identifier device", or "identifier and local data collector device" shown in FIG. 1 and FIG. 2.

In an embodiment, the animal further comprising a collar or a belt or a strap mounted to the animal.

The collar or belt or strap may be worn around a neck of the animal, or around a leg, or mounted to another suitable part of the animal. The collar or belt or strap may be attached in any suitable manner.

According to a fourth aspect, the present invention provides a farm comprising a plurality of animals according to the third aspect, and at least one beacon and the central data collector.

Preferably the farm comprises at least two, or at least three or at least four or at least five beacons.

According to a fifth aspect, the present invention provides a method of monitoring a living being using the monitoring sub-system of the first aspect, comprising the steps of: a) repeatedly retrieving temperature information from said temperature sensor; b) repeatedly retrieving identification information from said first non-volatile memory and/or medical information from the second memory; c) repeatedly retrieving signal strength information of the at least one beacon signal using the LF receiver; d) repeatedly transmitting said identifier information and said temperature information and said signal strength information and said identification information to a central data collector.

These steps are performed by the "identifier device" and the "local data collector" which may be implemented as a single module (in that case also referred to as "identifier and local data collector device" or as multiple modules, for example two modules, one of which may be implantable or implanted.

The "identification information" may be read-out repeatedly from the first non-volatile memory, but since the "identification data" does not change (during normal operation), it may also be read only once, and kept in a register or in RAM for example.

In an embodiment, the monitoring subsystem further comprises at least one sensor device, and the method further comprises the step of: repeatedly receiving via the wireless transceiver sensor information from the at least one sensor device; and step d) further comprises transmitting also said sensor information to the central data collector.

In an embodiment, the monitoring subsystem further comprises the central data collector, and the method further comprises the step of: estimating one or more physical locations of the living being based on at least one signal strength of at least one beacon signal.

This step is typically performed by the central data collector. If multiple beacons are arranged at predefined locations in and/or around a farm, the position of a particular animal can be rather precisely estimated, for example based on RSSI-data.

In an embodiment, the method further comprising the steps of: testing whether the temperature information is located in a first predefined value range; testing whether the sensor information is located in a second predefined value range; and providing an alarm signal if the outcome of the test is that the temperature information is located outside of the first predefined value range and/or the sensor information is located outside of the second predefined range.

Providing an alarm can for example be implemented by a showing of visual message on a screen connected to the central data collector, and/or by rendering an acoustic signal on the loudspeaker connected to the central data collector, or in any other suitable manner.

Particular and preferred aspects of the invention are set out in the accompanying independent and dependent claims. Features from the dependent claims may be combined with features of the independent claims and with features of other dependent claims as appropriate and not merely as explicitly set out in the claims.

These and other aspects of the invention will be apparent from and elucidated with reference to the embodiment(s) described hereinafter.

### Brief description of the drawings

**FIG**. **1** shows a high-level overview of a system for monitoring a plurality of animals living in or around a farm. The system comprises (inter-alia) devices mounted to the animal, capable of wirelessly communicating with (inter-alia) a readout device, beacons, and a central data collector. An important component for the present invention is the so-called "identification device". This component can be implanted in or attached to the animal.
   In the embodiment of FIG. 1, the identification device comprises a temperature sensor, a non-volatile memory for holding an identifier (ID) related to a specific animal, and for storing medical data of said animal, and comprises wireless communication means for retrieving data from one or more other sensors implanted in or attached to the animal. The identification device can be selectively programmed or updated or read out by means of a special readout device. The identifier device and the one or more sensors can be powered for example by a local battery, or wirelessly by means of electromagnetic waves sent by an energising unit which can be mounted for example in a collar worn by the animal.
**FIG**. **2** shows a variant of the system of FIG. 1, where the wireless communication means for retrieving data from the one or more other sensors is implemented in a device mounted in the collar. The other functionality of the "identifier device" remains the same as in FIG. 1.
**FIG**. **3** shows the "identifier and local data collector device" of FIG. 1 as an embodiment of the present invention, having two modes of operation: a "medical intervention mode" and a "normal mode of operation". The device is configured for interacting with a readout device when operating in the "medical intervention mode", and is adapted for interacting with one or more sensors, a central data collector, and one or more beacons when operating in the "normal operating mode".
**FIG**. **4** shows an example of an animal with the identifier device implanted or attached thereto, interfacing with a readout device, when operating in the "medical intervention mode".
**FIG**. **5** shows an example of an animal with the identifier device implanted or attached thereto, interfacing with a corresponding local data collector, with a plurality of beacons, and a plurality of sensor devices attached to and/or implanted in the animal, and a central data collector, when operating in the "normal operation mode".
**FIG**. **6** shows an exemplary hardware block diagram of an "identifier device" according to embodiments of the present invention.
**FIG**. **7(a) to FIG**. **7(c)** show a few examples of power supply means that may be used in sensor devices, and/or an identifier device, and/or a local data collector device as part of a system according to the present invention.
**FIG**. **8** shows an exemplary block diagram of a readout device as can be used in embodiments of the present invention.
**FIG**. **9** shows an exemplary block diagram of a sensor device, as can be used in embodiments of the present invention.

The drawings are only schematic and are non-limiting. In the drawings, the size of some of the elements may be exaggerated and not drawn on scale for illustrative purposes. Any reference signs in the claims shall not be construed as limiting the scope. In the different drawings, the same reference signs refer to the same or analogous elements.

### Detailed description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

In the present invention, the abbreviation "NV memory" means non-volatile memory.

In the present invention the term "confined environment" is used to indicate a limited or restricted area where animals can move, such as for example in one or a few meadows in relatively close vicinity to a barn.

In the present invention, the terms "identification device" and "identifier device" and "identifier module" are used as synonyms. This "identifier device" may optionally include a "local data collector" (as in FIG. 1), in which case also the term "identifier and local data collector device" is used to explicitly point out that the "identifier device" and the "local data collector device" are embedded in a single module. But the present invention will also work when the "identifier device" and the "local data collector device" are two separate, physically distinct devices or modules (as in FIG. 2), but adapted for cooperating and together performing a specific function as described herein.

When referring to "identifier device 110, 210", reference is made to the "identifier and local data collector device" 110 as shown in FIG. 1 or to the "identifier device" 210 shown in FIG. 2, but not necessarily to the (separate) local data collector device 220.

When reference is made to "ID" or "animal ID", what is meant is the identification code or identifier assigned to a specific living being, e.g. animal, which number or alphanumeric string is stored or is to be stored in a so called first non-volatile memory 602 of the "identifier device".

Where in the present invention reference is made to "registration of data", what is meant is that said data is stored in the non-volatile memory of the identifier device.

The present invention relates to the field of monitoring systems, in particular animal monitoring systems, or even more specifically to a system for monitoring a plurality of cows in a confined environment such as for example one or more meadows belonging to a particular farm.

Although the invention works for living beings of different kinds, for example poultry, mammals, in particular cattle, the invention will be described for the specific example of cows to simplify the description, but the present invention is not limited thereto. Likewise, in the detailed description it will be assumed that the "confined environment" comprises a barn and/or one or more meadows, but other confined environments are of course also possible, provided they can be surrounded by a reasonable number of beacons, for example less than 1000 beacons, or less than 250 beacons, or less than 50 beacons.

Although different wireless signals may be used, the present invention will be explained for beacons transmitting LF signals, and for the identifier device and/or local data collector communicating primarily via RF signals with other devices (e.g. readout device, sensors), and the optional energizing unit 125, 225 transmitting LF signals, but the present invention is not limited thereto, and other wireless signals may also be used.

**FIG**. **1** show a high-level overview of a complete system 100 for monitoring a plurality of cows 101 living and moving around in a farm, and in meadows around the farm. **FIG**. **2** shows a variant of the system 200, the only difference being that the "identifier module" and "local data collector" are implemented in a single module in system 100 of FIG. 1 but are implemented as two separate devices in the system 200 of FIG. 2.

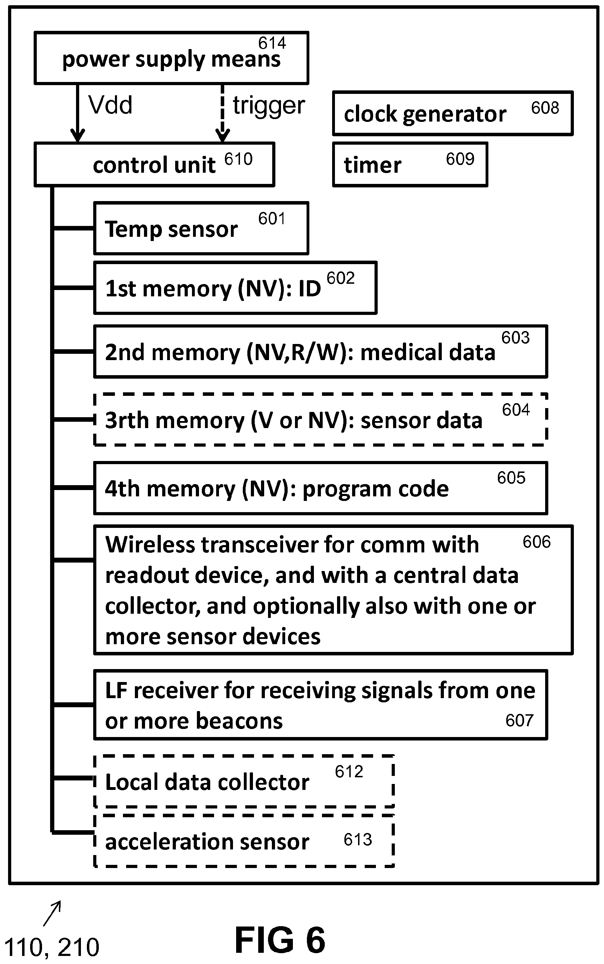

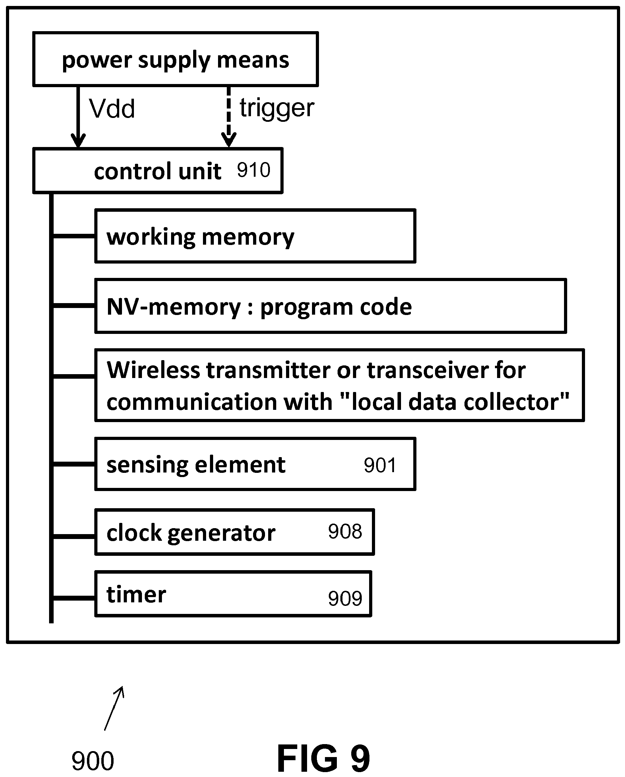

The total monitoring system 100, 200 including all optional devices, comprises:
(i) an "identifier module" 110, 210 containing inter-alia "identification information" allocated to an individual cow (e.g. a unique number for the cow within the farm, or even a unique number nationwide, or even a unique number worldwide), also referred to herein as the "identifier device". This module may be firmly attached to the cow, for example to one of its ears, on the inside or the outside of the ear, or may be implemented in a capsule which is implanted in the cow, for example shortly after its birth. It is intended that the identifier module 110 is permanently attached to or implanted in the individual cow for its entire lifetime. As will be explained further, this module 110, 210 also contains non-volatile memory 603 for storing a medical history of the specific animal 101, 201. This module furthermore contains a temperature sensor 601 and therefore needs to be in thermal contact with the animal. An exemplary hardware block diagram of an identifier module will be further described in relation to FIG. 6.
(ii) a "local data collector" which may be part 612 of the identifier module 110, or may be a separate module 220, adapted for cooperating with the identifier module 210. In the latter case, the "local data collector" is preferably also mounted to the cow, or simply carried by the cow, for example by being embedded in a collar worn placed around the neck of the cow, by attached to a leg of the cow by means of a strap or belt, or attached to the cow in any other suitable way. The "local data collector" need not be in direct physical contact with the animal.
(iii) optionally at least one additional sensor device 150, 250 different from the identifier module 110, 210, the additional sensor device also being attached to or implanted in the animal. The sensor device 150, 250 may, for example comprise a strain sensing element or an orientation sensing element or an acceleration sensing element or any other suitable sensing element. Importantly the sensor device 150, 250 also contains a processor for readout of the sensing element, and an wireless transmitter for repeatedly, for example periodically transmitting sensor information to the local data collector 110, 220. A possible hardware block diagram of a sensor device 150, 250 as can be used in the system 100 or 200, will be described in relation to FIG. 9. Such sensor devices are known per se in the art.
(iv) one or more beacons 130, 230 arranged at predefined locations of a confined environment, for example at different locations inside and/or outside a barn of a farm, and/or at different locations at the periphery of one or more meadows belonging to the farm. Each beacon 130, 230 transmits an LF signal having a carrier frequency in the range from about 50 kHz to 250 kHz. Typically multiple beacons are used, and they are distributed over the confined environment. By measuring for example the signal strength of the LF signals transmitted by the beacons, a position in the confined environment can be determined. Suitable beacons, and ways to determine a position relative to the beacons, based on the signal strength information, are known in the art, and hence need not be described in detail herein.
(v) a central data collector 170, 270 for example a standard computer provided with a suitable network card for receiving wireless RF signals, and provided with specific software for processing the data received from the one or more local data collectors 110, 220, typically one for each cow. The central data collector 170 may further comprise a monitor or a screen or a display for showing the information received from the plurality of local data collectors 110, 220, and/or may comprise input means such as a keyboard or a mouse or the like, for receiving input from a user, for example for receiving commands or queries from a user 180, e.g. from a farmer desiring to view sensor information and/or position information, and/or medical data related to his animals. The input and output devices are schematically represented by the block "UI" 171, 271.

### NORMAL OPERATION MODE:

During normal mode of operation, the above-mentioned devices, in particular: the identifier and local data collector device 110, or the identifier device 210 and the local data collector device 220 operating together, the sensors 150, 250, the beacons 130, 230 and the central data collector 170, 270 are cooperating to retrieve (inter-alia) temperature information and location information of each cow, and to repeatedly, e.g. periodically transmit the information of each cow to the central data collector 170, 270 which receives the information from each local data collector 110, 220, and typically stores the information in a database, e.g. on a hard disk on a computer, or on a network drive connected thereto, or a memory stick, etc. The central data collector 170, 270 typically also comprises software for extracting position information of each cow based on the signal strength information measured by each local data collector 120, 220 depending on its position relative to the beacons 130, 230, and since the positions of the beacons 130, 230 are fixed and known, the software can also estimate the physical position of each cow. Each cow may have a name, and the central data collector 170 can store a list of these names and the corresponding identification code "ID" as written in the identifier device 110, 210. The software on the central data collector 170 is preferably also capable of determining and/or showing the latest position information of a specific cow upon request, for example when the cows name or identification code ID is entered via an input means 171.

The devices involved in the normal mode of operation are also shown in **FIG**. 3, and in **FIG**. 5. In this mode, the identifier device 110, 210 acts primarily as a temperature sensor, capable of providing not only temperature information, but also the animal ID and medical information stored therein.

### MEDICAL INTERVENTION MODE:

Also shown in FIG. 1 and FIG. 2 is a readout device 140, 240. The readout device is not used during the normal operation mode, but is typically used by a veterinarian when doing a medical intervention. This mode of operation, also referred to herein as the "medical intervention mode", is also illustrated in FIG. 4, and involves in principle only the "identifier and local data collector device" 110 or the "identifier device" 210 on the one hand, and the readout device 140, 240 on the other hand. In this mode of operation, the "identifier and local data collector device" 110 or the "identifier device" 210 is operating in a second mode of operation different from the normal mode of operation.

In this second mode, the readout device 140, 240 can be used to program a newly defined identifier code (also referred to as "animal ID" or simply "ID") in a first non-volatile memory 602 (see FIG. 6) of the identifier device 110, 210. The first non-volatile memory is preferably a "One-Time-Programmable" non-volatile memory (also known as "OTP"), meaning that this memory can be written only once, and can be read multiple times, but cannot be overwritten or erased. It is an advantage that the ID cannot be erased, but remains the same for the cow during its entire lifetime. In this way, follow-up of the animal 101, 201 can be improved. The ID may be programmed in the identifier device 110, 210 for example shortly after birth of the animal, or shortly before or after the identifier device 110, 210 is attached to, or implanted in the animal.

Referring to **FIG**. **6**, the "identifier device" 110, 210 also contains a second non-volatile memory 603 for holding medical data of the cow, for example for storing the name and amount of medicines administrated to the cow, optionally combined with time and/or date information of administration, and optionally combined with an identification of the veterinarian, and optionally combined with textual comments from the veterinarian. This memory 603 can for example be implemented as standard flash or EEPROM. In some embodiments, the erase-function is enabled (for this second NV-memory 603, (in hardware and in software). In preferred embodiments however, the erase-function is disabled for this second NV-memory 603, in hardware and/or in software, but preferably in hardware. The latter provides the advantage that the medical history of a particular cow cannot be erased when the cow is sold to another owner, and allows improved control over medication administered to animals by authorities. Disabling the option to erase (and thus also to overwrite the data) in hardware provides better protection than an software solution, because the function cannot be maliciously enabled by a software-download for example.

Although described as "first memory" 602 and "second memory" 603, of course in practice this may be implemented as different address locations in a single OTP memory of sufficient size, e.g. at least 16 bytes, but preferably at least 256 bytes, or at least 1 KBytes, for example about 2 KBytes or about 4 KBytes.

The communication between the readout device 140, 240 and the identifier device 110, 210 may be encrypted using a secure protocol, e.g. based on AES-128. This further decreases the risk of unauthorised access and/or fraud.

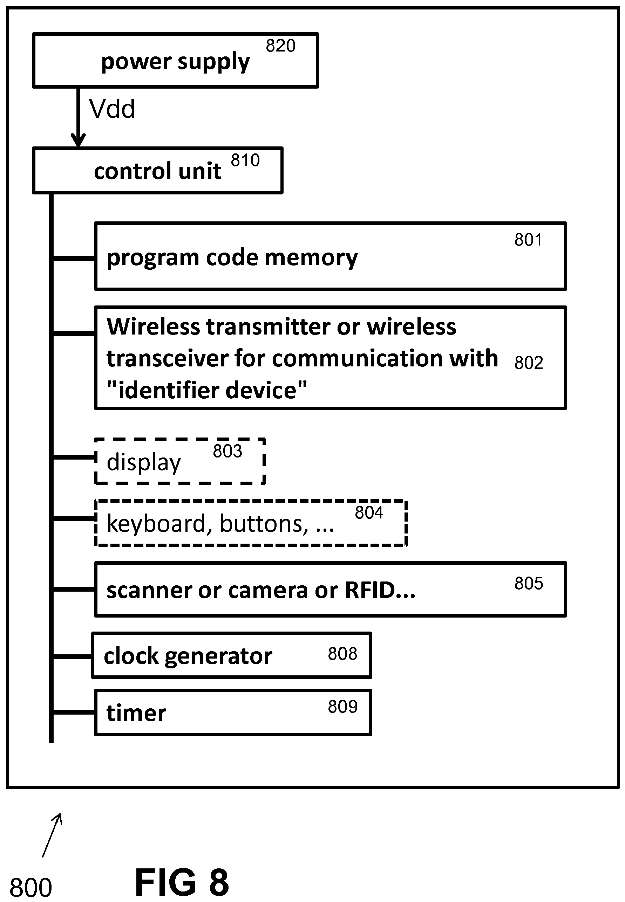

Envisioned readout devices 140, 240 are provided with a built-in camera or scanner or other recognition means 805 (see FIG. 8), and corresponding software stored in a program code memory 801 (e.g. flash memory) for recognising a "medicine ID" (e.g. a barcode or a two-dimensional code or the like) attached to or printed to a container of a particular medicine 190, 290 administered or to be administered to the cow. The veterinarian scans the code or barcode or color image or whatever kind of data carrier mounted on or printed on a medicine holder (e.g. box) 190, and the readout device 140, 240 converts this barcode or image into a numerical or alphanumerical string, referred to herein as "medical information", and preferably automatically transmits this medical information via a wireless transceiver 602, e.g. an RF transceiver to the "identifier device" 110, 210, without further intervention of the veterinarian. In this way human mistakes can be avoided as much as possible.

The medical data may optionally be accompanied by a time and date information (added by the readout device), and may optionally be accompanied by an identification of the veterinarian (added by the readout device), abbreviated herein as "vet ID". The veterinarian can optionally add textual comments via a user interface comprising for example a display 803 and a keyboard 804. Of course the latter can also be implemented as a virtual keyboard on a touch-screen (not shown) or the like. The identifier device 110, 210 receives the information via its wireless transceiver 606, e.g. RF transceiver, and adds the medical information and optional additional information to its second non-volatile memory 603.

Of course, the veterinarian can also consult medical information which was previously stored in the identifier device 110, 210. To this end, the readout device 140, 240 can send a request to the identifier device 110, 210 via wireless transceiver 802, which will receive the request via wireless transceiver 606, upon which request, the control unit 610 will readout at least a portion of the second memory 603, and transmit this data via wireless transceiver 606 to the wireless transceiver 802 of the readout device 140, 240.

As can be understood from the above, the identifier device 110, 210 is configured, when operating in the "medical intervention mode":
a) to receive an "animal ID" via its wireless transceiver 606, and to store the "animal ID" in the first NV-memory 602. (this can happen only once);
b) to receive a request (from a readout device) to read the animal ID (which is stored in the first NV-memory 602), and to transmit this animal ID via its wireless transceiver 606 (to the readout device 140, 240);
c) to receive a request (from a readout device 140, 240) to read at least a portion of the medical information stored in the second NV-memory 603, and to transmit this medical information via its wireless transceiver 606 (to the readout device 140, 240);
d) to receive a request (from a readout device 140, 240) to store additional medical information, to receive said medical information via the wireless transceiver 606 (from the readout device 140, 240), and to store, e.g. to add said medical data to the second second NV-memory 603.

As can be understood, the beacons 130, 230, and sensors 150, 250 and the local data collector 220, and the central data collector 170, 270 do not necessarily play a role in the "medical intervention mode". In this mode, the identifier device 110, 210 primarily acts as a wireless smart storage device.

### POWER SUPPLY:

A particular challenge related to the system 100, 200 is related to power supply of the various subsystems. This is not a problem for the central data collector 170, 270 and for the beacons 130, 230 which are typically powered via cable (e.g. connected to the mains supply). This is not really a problem for the readout device 140, 240 either, which is typically a portable device with a local battery, for example a replaceable and/or rechargeable battery. And also powering the local data collector 220 (see FIG. 2) is not a big challenge, as the local data collector 220 can for example be mounted to or integrated in a collar 211 worn by the animal, and this collar can also house a battery, which can easily be replaced, if needed. But a cable or a relatively thick battery is not the best solution for the sensors 150, 250 and for the "identifier device" 110, 210.

In embodiments of the present invention the sensors 150, 250 and/or the identifier device 110, 210 have at least one local non-rechargeable battery, see **FIG**. **7(a)****.** A disadvantage of this solution is that the battery may not have sufficient energy for the entire lifetime of the animal, and hence the sensor and/or the battery needs to be replaced after some time.

In other embodiments of the present invention the sensors 150, 250 and/or the identifier device 110 have at least one local rechargeable battery or capacitor, as shown for example in **FIG**. **7(b)****,** and a contactless recharging means. The latter may for example comprise a coil adapted for receiving electromagnetic energy from LF or RF radiated electromagnetic waves, and/or may for example comprise one or more solar cells, or any other means for receiving electromagnetic energy in a contactless manner. An advantage of such solution is that the battery or capacitor can be recharged multiple times during the lifetime of the animal, and hence the device need not be removed.

In further or other embodiments of the present invention the sensors 150 and/or the identifier device 110 may have at least one local rechargeable battery or capacitor and an energy harvesting means, as shown for example in **FIG**. **7(c)****.** The latter may for example comprise circuitry for converting thermal energy into electric energy, for example by means of one or more thermopiles, or may for example comprise circuitry for converting mechanical energy into electric energy. In this way thermal heat or kinetic energy generated by the cow may can be converted into electrical energy, which can be used to recharge the battery or capacitor. In all of the embodiments, additional circuitry such as a voltage multiplier and/or a DC/DC convertor and the like may also be used. Recharging circuits are known per se in the art and hence need not be explained in detail herein.

It is noted that the same or different powering solutions can be chosen for the one or more sensors 150, 250 and for the identifier andr local data collector device 110 or for the the identifier device 210 and the local data collector device 220.

Referring back to **FIG. 1** and **FIG**. **2**, according to a particular embodiment of the present invention, the system further comprises an energiser module 125, 225, adapted for transmitting electromagnetic energy at LF frequency, in order to provide power the one or more sensors 150 and/or the identifier device 110, 210, as indicated by the arrows "LF-E" (indicating radiated Energy in the form of electromagnetic waves at LF frequency). The energizer module may for example be embedded in or housed by a collar 111, 211 or a strap or a belt or the like. The energizer module 125, 225 itself would typically have a relatively large and rechargeable and/or replaceable battery (e.g. having a capacity of at least 5000 mAh or at least 10000 mAh), which could be recharged or replaced during the night in any suitable manner.

Instead of, or in addition to charging/recharging the sensors 150, 250 and/or the identifier device 110, 210 out in the field by means of electromagnetic low-frequency waves "LF-E", transmitted by an energiser module 125, 225, the sensors and the identifier device may also be recharged during the night, when the animal is in the stable or the barn or the like, via energizer modules (not shown) mounted in the barn, connected to the mains supply, likewise transmitting LF-E signals.

For completeness it is noted that the arrows "RF-d" are also electromagnetic waves but at an RF frequency and they are modulated to transfer data. The arrows "LF-d" transmitted by the beacons are also electromagnetic waves at LF frequency, but also these are modulated to carry some form of data for example a beacon identification code. In preferred embodiments, the energising waves "LF-E" transmitted by the energiser module 125, 225 are un-modulated waves, e.g. sinusoidal waves.

Another aspect related to saving energy is that the sensor devices 150, 250 and the "identifier and local data collector device" 110 (of FIG. 1) and the "identifier device" 210 and the "local data collector device" 220 (of FIG. 2) are also provided with a timer 609, and are capable of going into low power mode (e.g. sleep mode or standby-mode) and to periodically wake up, to quickly measure something, transmit this information, and go back to sleep.

The idea is that, during normal operation mode, the one or more sensors 150, 250 would periodically wake up, obtain sensor information from its sensor element, and transmit that sensor information via a wireless signal to the local data collector 220. The local data collector 220, or if embedded in the same device as the identifier device, the "identifier and local data collector device" 110 would wake up at about the same time and would be able to receive the sensor information transmitted by the one or more sensors 150, 250. The local data collector 220 would also receive or retrieve the temperature information and/or identification information from the "identification device" 110, 210, and would retrieve or receive signal strength information related to the beacon signals, and would combine this information in a data package, and transmit the data package to the central data collector 170, 270 via its wireless transceiver 606.

By providing sensor devices 150, 250 with a sleep mode, a considerable amount of energy can be saved. Furthermore, as the sensors 150, 250 only need to transmit over a very short distance , namely to the local data collector 220, which is typically less than 300 cm or even less than 200 cm away from the sensor, the power at which the sensor devices 150 need to transmit can be drastically reduced, which further reduces the average power consumption. The same is true for the identifier device 110 in the form of a discrete device excluding the data collector device 220, when it transmits the temperature information and/or identification information to the local data collector.

In contrast, the local data collector device 220, - or in case the local data collector is integrated in the same module as the identifier device, the integrated identifier device 110 -, needs to transmit the information over a relatively large distance (e.g. up to 100 m or up to 200 m) to the central data collector 170, 270 and therefore needs a more powerful battery. It is advantageous that only one device (namely the local data collector) needs to transmit over a relatively large distance, rather than multiple devices having to do so. This is considered an important advantage of at least some embodiments of the present invention.

As an optional further improvement, it is noted that the sensor information and the temperature information need not be sampled and transmitted at the same rate as to beacon information. In preferred embodiments of the present invention, the local data collector 170, 270 samples or determines the signal strength information related to the beacon signals at a first frequency F1, and retrieves or receives or requets the sensor information and/or the temperature information at a second frequency F2 lower than the first frequency F1. The ratio R of the first frequency F1 and the second frequency F2, is preferably an integer value N in the range from 1 to 10, for example equal to 2 or 3 or 4. Expressed in mathematical form: R=F1/F2=N, N being an integer. Choosing an integer value for the ratio R allows that 1 out of N transmissions can contain both beacon information and sensor information and temperature information and animal-ID-information, while

(N-1) out of N transmissions contain only beacon information plus animal-ID-information, and optionally also temperature information, but no sensor information from the additional sensors 150, 250. Using a higher sampling and transmission rate for the beacon information than for the sensor information offers the advantage of being able to update the location information faster than the sensor information. This combines the advantages of being able to track the location of the animals more closely, while at the same time saving energy for the sensor devices, which allows to decrease the battery capacity and thus battery size and weight, and thus the cost and size of the sensors or sensor modules (including such battery), which makes mounting or attachment or implantation of such sensors easier. This advantage should not be underestimated.

As already indicated above, **FIG**. **2** shows a variant of the system of FIG. 1, where the identifier device 210 and the local data collector device 220, are separate units or modules. The identifier device 210 needs to be in physical contact with the animal, for example by attachment or implantation in the animal, preferably for its entire lifetime, while the local data collector device 220 does not need to be in fysical contact with the animal, and may be worn around the neck or a leg of the animal, for example embedded in a collar or a strap or a belt or the like.

Apart from this difference, the system 100 of FIG. 1 and the system 200 of FIG. 2 work in much the same way, and therefore the rest of the invention will only be described further for the monitoring system 100 shown in FIG. 1, but the invention is not limited thereto, but only by the claims.

**FIG**. **3** shows the "identifier and local data collector device" 110 of FIG. 1 as an embodiment of the present invention. As already described above, the device 110 has two modes of operation: a "medical intervention mode" and a "normal mode of operation". The device 110 is configured for interacting via its wireless transceiver 606 (see FIG. 6) with a readout device 140, when operating in the medical intervention mode. The device 110 is further configured for interacting via its wireless transceiver 606 with a central data collector 170 and optionally also with with one or more sensors 150, and for receiving (via its LF receiver 607) LF-signals from one or more beacons 130, when operating in the normal operating mode.

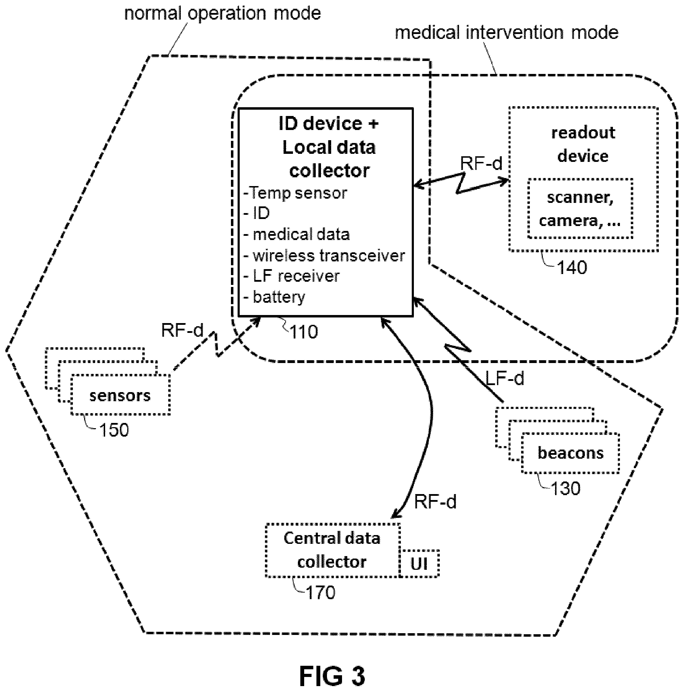

While FIG 3 shows the identifier and local data collector device 110 of the system of FIG. 1, a similar picture (not shown) can be provided where the device 110 is replaced by the identifier device 210 plus the local data collector device 220 of FIG. 2, working together. It does not matter for the present invention whether these devices are physically integrated in a single device 110, or whether the functionality is split over two (or more) devices 210, 220 offering the same functionality. That is a matter of implementation.

Taking into account that the identifier device 110, 210 further includes a temperature sensor 601, and a first and second non-volatile memory 602, 603, and wireless transceiver 606 (e.g. an RF transceiver), and an LF receiver, it can be stated that FIG. 3 shows a monitoring subsystem 110; 210, 220 attachable to or at least partly implantable in a living being, for example a cow, the monitoring subsystem comprising: a temperature sensor 601 (see also FIG. 6); a first non-volatile memory 602 for holding an identification information (animal-ID) to be associated to said living being; a second non-volatile memory 603 for holding medical information related to said living being; a wireless transceiver 606; an LF receiver 607 for receiving at least one beacon signal transmitted by at least one beacon, and comprising means for measuring a signal strength of said at least one beacon signal; a control unit 610 operatively connected to the temperature sensor 601, and to the first and second non-volatile memory 602, 603 and to the wireless transceiver 606 and to the LF receiver 607, and being programmed for communicating via the wireless transceiver with a readout device 140, 240 for exchanging the animal-ID and medical information in a first mode of operation (also referred to herein as "medical intervention mode"), and optionally being programmed for repeatedly communicating via the wireless transceiver 606 with one or more sensor devices 150, 250 for receiving sensor information, and being programmed for repeatedly retrieving temperature information from the temperature sensor 601, and for repeatedly retrieving signal strength information from the LF receiver 607 indicative of a distance to one or more beacons 130, and for repeatedly transmitting said temperature information and signal strength information and identification information and optionally also said sensor information via the wireless transceiver 606 to a central data collector 170, in a second mode of operation (also referred to herein as "normal operation mode").

**FIG**. **4** shows an example of an animal with the identifier device 210 implanted or attached thereto. The identifier device 210 is adapted for communicating (directly or indirectly) with a readout device 140, for example during a medical intervention, to illustrate a first mode of operation of the identifier device, also referred to herein as the "medical intervention mode".

Actually the "medical intervention mode" can be subdivided in three modes:
(a) a first medical intervention mode, also referred to herein as initialization mode, where a new identifier, e.g. a alphanumerical string is sent by the readout device 140 to be programmed in the first non-volatile memory 602 of the identification device 110, 210. In this mode also medical data can be sent to the device 110, 210 which will store said medical data in its second non-volatile memory 603;
(b) a second medical intervention mode, in which mode the identifier information can be read from the first non-volatile memory 602, and/or medical information can be read from the second non-volatile memory 603, and/or additional medical data can be written to the second non-volatile memory 603. The additional medical data can for example be automatically transmitted by the readout device 140 after scanning a medical label comprising for example a barcode or a QR code, or any other kind of one-dimensional or two-dimensional code. To this end the readout device 140 has a scanner 805 (see FIG. 8), for example a laser scanner or an imager and a corresponding data processing module to convert the scanned information into an alphanumerical string;
(c) a programming mode, in which mode the identifier device may be adapted for accepting new software (from the readout device) and for storing it in the program memory 605, and/or may be adapted for receiving one or more configurable settings (from the readout device), such as for example the ratio R mentioned above, a value indicative of how long to stay awake for receiving sensor information, sleep time, listening time, etc.

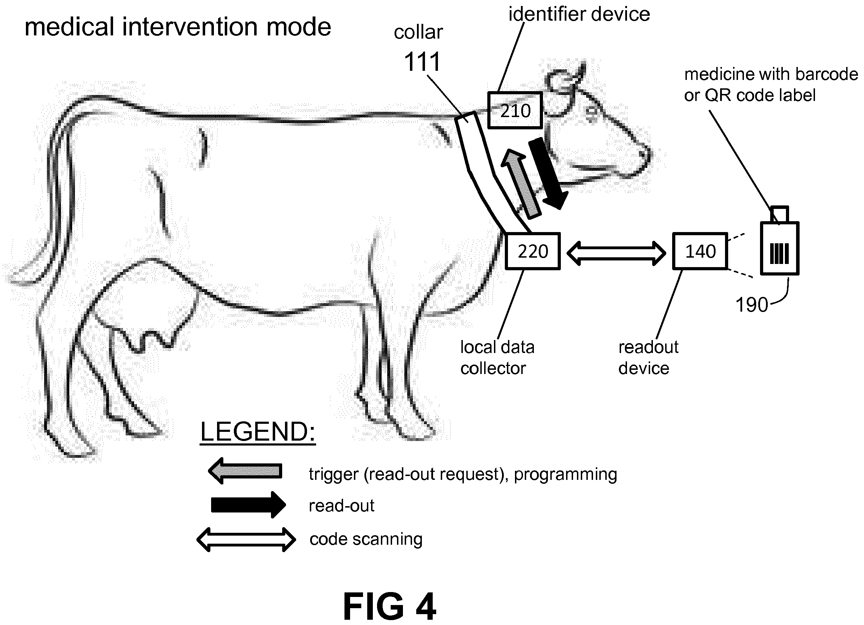

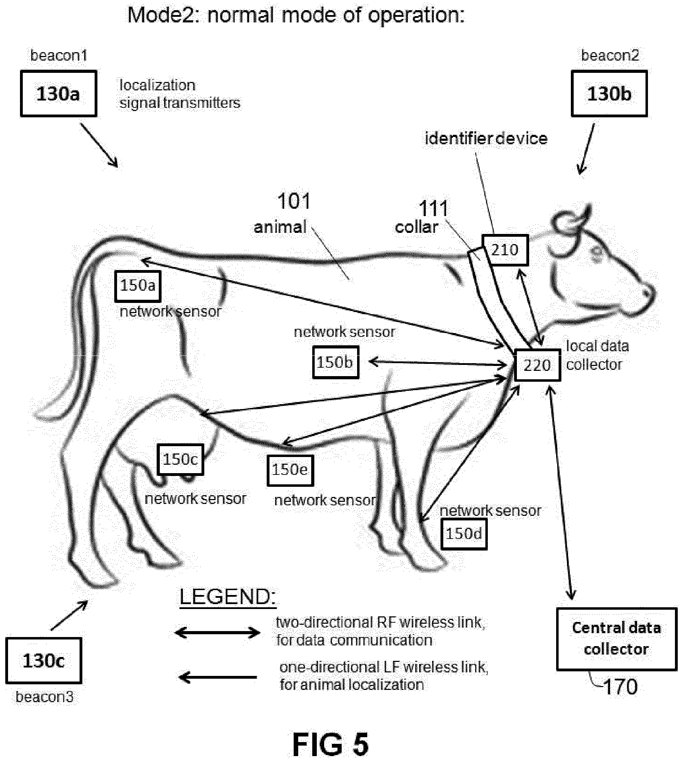

In the example shown in FIG. 4, the readout device 140 does not communicate directly to the identifier device 210, but indirectly via the local data collector 220. In an alternative embodiment, the readout 140 communicates directly with the identifier device 210.

In yet another embodiment, where the identifier device 110 is of the kind as shown in FIG. 1, the readout device 140 also communicates directly with the "identifier device".

**FIG**. **5** shows an example of an animal 101 with the identifier device 210 implanted or attached thereto, and comprising, e.g. carrying a local data collector 220 (e.g. mounted in a collar 111, or in a strap or belt or in any other way), which local data collector 220 is adapted for communicating with a central data collector 170, the beacons 130, and the central data collector 170, when operating in the second mode of operation. The local data collector of FIG. 4 is also adapted for interacting with the one or more sensor devices 150, mounted on or implanted in the animal 101, and for transmitting the sensor information to the central data collector 170. In this way, the power required by the sensor devices 150 can be drastically reduced.

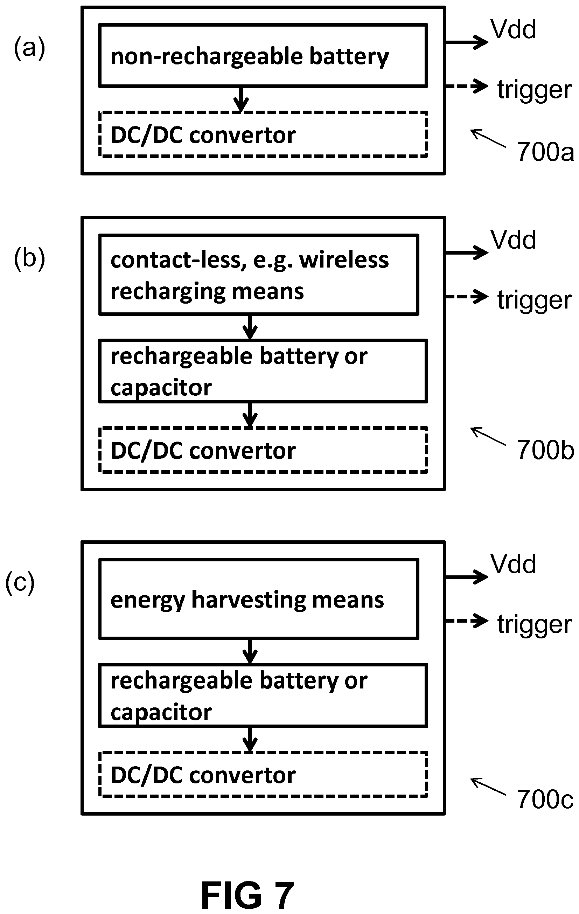

**FIG**. **6** shows an exemplary hardware block diagram of an "identifier device" 210 or an "identifier and local data collector device" 110 according to embodiments of the present invention, depending on whether the local data collector 612 is embedded in the identifier device or not. As shown, the device of FIG. 6 may also comprise a third memory 604, for example volatile memory such as e.g. RAM or registers for temporarily storing sensor information retrieved from the data sensors. The control unit 610 is preferably a programmable processor, programmed with an algorithm which is stored in a non-volatile memory 605 containing program code. The first and/or second and/or third and/or fourth non-volatile memory may be implemented as non-overlapping memory-space of a single non-volatile memory, but that is not absolutely required. As described above, preferably the first non-volatile memory, where the ID is written, is OTP memory space. The device 110, 210 further comprises a clock generator 608, for example based on an RC oscillator, and a timer 609 with wake-up functionality. The device 110, 210 may also comprise a motion sensor, and/or an orientation sensor, e.g. comprising an accelerometer, and/or gyroscope and/or a magnetometer, for allowing tracking of movements and/or physical activities of the cow. The unit also comprises some kind of power supply means 614, a few possible examples of which are shown in FIG. 7.

**FIG**. **7(a) to FIG. 7(c)** shows an a few examples of power supply means that may be used in conjunction with the sensor devices 150, and in conjunction with the identifier device 110, 210, but the present invention is not limited to these implementations and other means of providing power can also be used. The functioning of these examples was already described above, and will not be repeated here. In the example of FIG. 7(b), the local battery or capacitor is preferably dimensioned for providing sufficient energy for a predefined number of days, for example at least 1 day, or at least 2 days, or at least 3 days without being recharged. Although not explicitly shown, the battery-powered devices may also have a low voltage indicator, and the low-voltage information may also be transmitted to the local data collector 210 and to the central data collector 170 to indicate a low power state.

**FIG**. **8** shows an exemplary block diagram of a readout device 140, 240 as can be used in conjunction with the identifier device 110, 210, but the present invention is not limited thereto and other readout devices may also be used. It was already explained above how this device works, in particular in conjunction with the identifier device.

**FIG**. **9** shows an exemplary block diagram of a sensor device 150, 250, as can be used in embodiments of the present invention, but the present invention is not limited thereto and other sensor devices may also be used. The sensing element 901 may for example be a motion sensor, or an orientation sensor, or a stress sensor, or a temperature sensor, or a pH sensor, or a liquid sensor, or any other kind of sensor. It is already explained above how such a sensor would operate in embodiments of the present invention. For example, the control unit 910 is programmable, and has a low-power mode, and has a clock generator 908 and a timer 909, etc.

## Claims

1. A monitoring subsystem (110; 210) attachable to or at least partly implantable in a living being (101; 201), the subsystem comprising:
- a temperature sensor (601);
- a first non-volatile memory (602) for holding an identification information (ID) to be associated to said living being;
- a second non-volatile memory (603) for holding medical information related to said living being;
- an wireless transceiver (606);
- an LF receiver (607) for receiving at least one beacon signal transmitted by at least one beacon, the LF receiver comprising means for measuring a signal strength of said at least one beacon signal;
- a control unit (610) connected to the temperature sensor (601), and to the first and second non-volatile memory (602, 603) and to the wireless transceiver (606) and to the LF receiver (607),
- the control unit being programmed for communicating via the wireless transceiver (606) with a readout device (140; 240) for exchanging the identification information (ID) and/or for exchanging medical information in a first mode of operation;
- and the control unit being programmed for repeatedly retrieving temperature information from the temperature sensor (601), and for repeatedly retrieving signal strength information from the LF receiver (607) indicative of a distance to one or more beacons (130, 230), and for repeatedly transmitting said temperature information and said signal strength information and said identification information and optionally the medical information via the wireless transceiver (606) to a central data collector (170, 270) in a second mode of operation.

2. A monitoring subsystem (110; 210) according to claim 1, wherein the control unit (610) is further programmed for repeatedly communicating via the wireless transceiver (606) with one or more sensor devices (150, 250) attached to or implanted in said living being (101; 201), for receiving sensor information, and for transmitting said sensor information to the central data collector (170; 270).

3. A monitoring subsystem (110; 210) according to claim 1 or 2, implemented in a single module or capsule.

4. A monitoring subsystem (110; 210) according to claim 1 or 2, implemented as more than one module, comprising at least:
- a first module (210) comprising at least the temperature sensor (601), and the first non-volatile memory (602), and the second non-volatile memory (603), and the wireless transceiver (606) for communication with the readout device, and for communication with a local data collector (220) if not embedded in the subsystem, and a first local power supply (614);
- a second module (220) different from the first module (210), comprising at least the LF receiver (607), and a second wireless transceiver for communication with the central data collector (170; 270) and optionally for communication with the one or more sensor devices (150; 250), and a second local power supply, and optionally an acceleration sensor.

5. A monitoring subsystem (110; 210) according to any of the previous claims, further comprising:
- a local energising unit (125; 225) for providing power to the identifier device (110; 210) in a wired or wireless manner.

6. A monitoring subsystem (110; 210) according to any of the previous claims, further comprising at least one sensor device (150; 250) attached to or implanted in the living being (101; 201) for measuring a feature or characteristic of the living being.

7. A monitoring subsystem according to claim 6,
wherein the at least one sensor device (150; 250) is a sensor selected from the group consisting of: a strain sensor, a pressure sensor, a milk sensor, a liquid sensor, a PH-sensor, a blood analysis senor, a sugar sensor, a rumination sensor or gas sensor or SCFA sensor;
and wherein the at least one sensor comprises a wireless transmitter or a wireless transceiver (802) and is adapted for transmitting sensor information to the subsystem (110; 210, 220).

8. A monitoring subsystem according to any of the previous claims,
further comprising at least one beacon (130; 230),
and/or further comprising the central data collector (170; 270).

9. A system comprising:
- at least one or at least two monitoring subsystems according to any of the claims 1 to 7;
- at least two beacons (150; 250);
- at least one central data collector (170; 270).

10. A monitoring subsystem according to any of claims 1 to 8 or a system according to claim 9,
- further comprising a readout device (140; 240), the readout device further comprising means for obtaining medical information of a medical substance administered to or to be administered to the living being (101; 201), the readout device being adapted for transmitting said medical information to the subsystem;
- the monitoring subsystem being further adapted for storing said medical information in the second non-volatile memory (603).

11. An animal (101; 201) comprising a monitoring subsystem according to any of the claims 1 to 7, and optionally further comprising a collar or belt or strap (111; 211) mounted to the animal.

12. A method of monitoring a living being (101; 201) using the monitoring sub-system of any of the claims 1 to 7, comprising the steps of:
a) repeatedly retrieving temperature information from said temperature sensor (601);
b) repeatedly retrieving identification information (ID) from said first non-volatile memory (602) and/or medical information from the second memory (603);
c) repeatedly retrieving signal strength information of the at least one beacon signal (150; 250) using the LF receiver (607);
d) repeatedly transmitting said identifier information and said temperature information and said signal strength information and said identification (ID) information to a central data collector (170; 270).

13. The method of claim 12,
wherein the monitoring subsystem further comprises at least one sensor device (150; 250); and wherein the method further comprises the step of:
- repeatedly receiving via the wireless transceiver (608) sensor information from the at least one sensor device (150; 250);
- and wherein step d) further comprises transmitting also said sensor information to the central data collector (170; 270).

14. A method according to claim 12 or 13,
wherein the monitoring subsystem further comprises the central data collector (170; 270), and wherein the method further comprises the step of:
- estimating one or more physical locations of the living being (101; 201) based on at least one signal strength of at least one beacon signal (150; 250).

15. A method according to any of the claims claim 12 to 14, further comprising the steps of:
- testing whether the temperature information is located in a first predefined value range,
- testing whether the sensor information is located in a second predefined value range,
- and providing an alarm signal if the outcome of the test is that the temperature information is located outside of the first predefined value range and/or the sensor information is located outside of the second predefined range.
